(19) Europäisches Patentamt
European Patent Office
Office européen des brevets

(11) **EP 2 068 972 B1**

## (12) EUROPÄISCHE PATENTSCHRIFT

(45) Veröffentlichungstag und Bekanntmachung des Hinweises auf die Patenterteilung:
**06.11.2013 Patentblatt 2013/45**

(21) Anmeldenummer: **07818382.9**

(22) Anmeldetag: **25.09.2007**

(51) Int Cl.:
***A61M 1/16*** *(2006.01)*

(86) Internationale Anmeldenummer:
**PCT/EP2007/008297**

(87) Internationale Veröffentlichungsnummer:
**WO 2008/037410 (03.04.2008 Gazette 2008/14)**

(54) **VORRICHTUNG UND VERFAHREN ZUR VORGABE EINER DIALYSIERFLÜSSIGKEITSRATE ODER BLUTFLUSSRATE FÜR EINE EXTRAKORPORALE BLUTBEHANDLUNG**

DEVICE AND METHOD FOR DETERMINING A DIALYSIS FLUID FLOW RATE OR BLOOD FLOW RATE FOR EXTRACORPOREAL BLOOD TREATMENT

PROCÉDÉ ET DISPOSITIF DE DÉFINITION D'UN DÉBIT DE LIQUIDE DE DIALYSE OU D'UN DÉBIT SANGUIN POUR UN TRAITEMENT SANGUIN EXTRACORPOREL

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.09.2006 DE 102006045437**

(43) Veröffentlichungstag der Anmeldung:
**17.06.2009 Patentblatt 2009/25**

(73) Patentinhaber: **Fresenius Medical Care Deutschland GmbH**
**61352 Bad Homburg v.d.H. (DE)**

(72) Erfinder:
• **MOISSL, Ulrich**
**61118 Bad Vilbel (DE)**
• **WÜPPER, Andreas**
**64572 Büttelborn (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
**US-A- 5 092 836**

## Beschreibung

Die Erfindung bezieht sich auf eine Vorrichtung und ein Verfahren zur Vorgabe einer Dialysierflüssigkeitsrate oder Blutflussrate für eine extrakorporale Blutbehandlungsvorrichtung, die einen Dialysator aufweist, der durch eine semi-permeable Membran in eine Blutkammer, die von Blut mit einer vorgegebenen Blutflussrate durchströmt wird, und eine Dialysierflüssigkeitskammer unterteilt ist, die von Dialysierflüssigkeit mit einer vorgegebenen Dialysierflüssigkeitsrate durchströmt wird. Darüber hinaus betrifft die Erfindung eine Blutbehandlungsvorrichtung mit einer Vorrichtung zur Vorgabe einer Dialysierflüssigkeitsrate oder Blutflussrate und ein Verfahren zum Betreiben einer extrakorporalen Blutbehandlungsvorrichtung.

Bei Verfahren der Blutreinigungstherapie wie Hämodialyse, Hämofiltration und Hämodiafiltration wird Blut eines Patienten über einen extrakorporalen Blutkreislauf geleitet, in dem sich ein Dialysator bzw. Filter befindet, der durch eine semipermeable Membran in eine Blutkammer und eine Dialysierflüssigkeitskammer bzw. Filtratkammer getrennt ist. Bei einer Hämodiafiltration wird sowohl eine Hämodialyse als auch eine Hämofiltration durchgeführt. Die Erfindung bezieht sich auf alle Verfahren der Blutreinigungstherapie, bei denen Blut durch die Blutkammer und Dialysierflüssigkeit durch die Dialysierflüssigkeitskammer eines Dialysators strömt.

Es sind verschiedene physikalische und/oder chemische Größen bekannt, mit denen die Leistungsfähigkeit des Dialysators und/oder die Effektivität einer Dialysebehandlung angegeben werden können. Eine bekannte Größe für die Leistungsfähigkeit eines Dialysators stellt die Clearance K dar. Die Clearance K einer Substanz ist der Teilstrom des Gesamtstroms durch den Dialysator, der von der betreffenden Substanz vollständig befreit wird. Für die Effektivität einer Dialysebehandlung ist von entscheidender Bedeutung die sogenannte Dialysedosis KT/V, die als der Quotient aus dem Produkt von Clearance K für Harnstoff und effektiver Behandlungszeit T der Dialysebehandlung und dem Verteilungsvolumen V des Patienten für Harnstoff definiert ist.

Die US 5,100,554 beschreibt ein Verfahren zur Bestimmung der Clearance, bei dem der Dialysator-Elektrolyttransfer jeweils bei zwei unterschiedlichen Dialysateingangskonzentrationen gemessen wird. Aus der US 5,100,554 ist bekannt, dass die Effektivität der Dialysebehandlung von dem Blutfluss und dem Dialysierflüssigkeitsfluss abhängig ist.

Die DE 695 31 137 T2 (WO 95/32010) beschreibt ein Verfahren und eine Vorrichtung zur Optimierung der Effektivität einer Dialysebehandlung, wobei eine für die Effektivität der Dialysebehandlung charakteristische Größe während der Behandlung gemessen und auf der Grundlage der gemessenen Größe ein Parameter der Dialysebehandlung bestimmt wird, um eine optimale Effektivität der Dialysebehandlung zu erzielen. Der Druckschrift ist im Einzelnen zu entnehmen; dass ein Prozessor die gewählten Parameter, beispielsweise die Blutflussrate schrittweise in definierten Inkrementen erhöht, wobei ein Harnstoff-sensor eine charakteristische Größe fortlaufend misst, in dem eine Probe des ablaufenden Dialysats genommen wird. Durch Vergleich der aktuell gemessenen Konzentration mit der vorhergehenden Konzentration wird ermittelt, ob die aktuelle Konzentration kleiner oder größer als die vorhergehende Konzentration ist. Wenn die aktuelle Konzentration kleiner als die vorhergehende Konzentration ist, wird darauf geschlossen, dass die vorhergehende Konzentration den optimalen Wert darstellt. Folglich soll der Maximalwert der Konzentration bestimmt werden.

In der Vergangenheit sind die bekannten Dialysevorrichtungen mit einem konstanten Dialysierflüssigkeitsfluss, der vom Benutzer nicht veränderbar war, betrieben worden. Neuere Geräte erlauben hingegen die manuelle Einstellung unterschiedlicher Dialysierflüssigkeitsraten, beispielsweise 300, 500 und 800 ml/min. Zur Erzielung einer hohen Clearance sind grundsätzlich höhere Dialysierflüssigkeitsflüsse bei höheren Blutflüssen erforderlich.

Bei der Einstellung eines bestimmten Dialysierflüssigkeitsflusses ist zu berücksichtigen, dass mit einem hohen Dialysierflüssigkeitsfluss zwar eine hohe Clearance erzielt werden kann, sich aber die Kosten für die Bereitstellung frischer und die Entsorgung verbrauchter Dialysierflüssigkeit erhöhen. Daher wird in der Praxis eine verhältnismäßig hohe Clearance bei relativ niedrigem Verbrauch an Dialysierflüssigkeit angestrebt.

Es ist bekannt, dass unter Verwendung der weit verbreiteten Dialysatoren bei einem Verhältnis von Blutfluss zu Dialysierflüssigkeitsfluss von 1:2 eine gegenüber einem nicht veränderbaren Dialysierflüssigkeitsfluss von 500 ml/min nur kleine Verringerung der Clearance auftritt (J.E. Siegdell, B. Tersteegen, Artificial Organs, 10(3) Seiten 219 bis 225, 1986).

Die US 5,092,836 schlägt daher vor, den Dialysierflüssigkeitsfluss in Abhängigkeit vom Blutfluss nach vorgegebenen Kriterien zu steuern. Es wird insbesondere vorgeschlagen, einen Dialysierflüssigkeitsfluss einzustellen, der sich durch Multiplikation des Blutflusses mit einem konstanten Faktor ergibt. Neben einem linearen Zusammenhang zwischen Blut- und Dialysierflüssigkeitsfluss wird ein numerisches Datenfeld vorgeschlagen, das zu jedem Blutfluss eines bestimmten Dialysators denjenigen Dialysierflüssigkeitsfluss angibt, bei dem ein bestimmter Prozentsatz der maximalen Clearance, die unter der Annahme eines unendlich hohen Dialysierflüssigkeitsflusses vorliegen müsste, erreicht wird. In der Praxis kann der Prozentsatz beispielsweise bei 95 % liegen.

Der Erfindung liegt die Aufgabe zu Grunde, eine Vorrichtung und ein Verfahren zur Vorgabe einer optimalen Dialysierflüssigkeitsrate oder Blutflussrate für eine extrakorporale Blutbehandlungsvorrichtung anzugeben, wobei einerseits der Forderung nach einer hohen Effektivität der Dialysebehandlung und andererseits nach einem geringen Verbrauch von Dialysierflüssigkeit Rechnung getragen wird. Eine weitere Aufgabe der Erfindung liegt darin, eine Blutbehandlungsvorrichtung bereitzustellen, mit der eine Dialysebehandlung mit verhältnismäßig hoher Effektivität bei einem relativ geringem Dialysierflüssigkeitsfluss durchgeführt werden kann. Eine Aufgabe der Erfindung ist auch, ein Verfahren zum Betreiben einer Blutbehandlungsvorrichtung anzugeben, um eine Dialysebehandlung mit einer relativ hohen Effektivität bei einem angemessenen Verbrauch von Dialysierflüssigkeit durchführen zu können.

Die Lösung dieser Aufgaben erfolgt erfindungsgemäß mit den Merkmalen der Patentansprüche 1, 8 und 11. Vorteilhafte Ausführungsformen der Erfindung sind Gegenstand der Unteransprüche.

Bei der erfindungsgemäßen Vorrichtung und dem erfindungsgemäßen Verfahren zur Vorgabe einer Dialysierflüssigkeitsrate bzw. Blutflussrate für eine extrakorporale Blutbehandlungsvorrichtung wird bei einer vorgegebenen Blutflussrate diejenige Dialysierflüssigkeitsrate $Q_d$ ermittelt, bei deren Erhöhung um einen bestimmten Wert die Erhöhung einer für die Effektivität der Blutbehandlung charakteristischen Größe einen bestimmten Wert nicht unterschreitet. Alternativ kann auch bei einer vorgegebenen Dialysierflüssigkeitsrate diejenige Blutflussrate $Q_b$ ermittelt werden, bei deren Erhöhung um einen bestimmten Wert die Erhöhung einer für die Effektivität der Blutbehandlung charakteristischen Größe einen bestimmten Wert nicht unterschreitet.

Die erfindungsgemäße Vorrichtung und das erfindungsgemäße Verfahren gehen dabei davon aus, dass ab einem optimalen Wert für die Dialysierflüssigkeitsrate bei einer vorgegebenen Blutflussrate bzw. für die Blutflussrate bei einer vorgegebenen Dialysierflüssigkeitsrate zwar mit einer weiteren Erhöhung der Dialysierflüssigkeitsrate bzw. Blutflussrate noch eine Erhöhung der Effektivität der Dialysebehandlung erzielt werden kann, die zusätzliche Dialysierflüssigkeit bzw. die weitere Erhöhung des Blutflusses, die für eine derartige Behandlung mit höherer Effektivität erforderlich ist, aber nicht in einem wirtschaftlichen Verhältnis zu der damit verbundenen Erhöhung der Effektivität steht. Es wird also als Zielkriterium der Arbeitspunkt gesucht, bei dem der Verbrauch an zusätzlicher Dialysierflüssigkeit, der für die Erhöhung der Clearance um einen bestimmten Wert erforderlich wäre, einen bestimmten Wert nicht überschreitet, d.h. es wird ermittelt, wie viel ml/min Dialysierflüssigkeit man bereit ist zu verbrauchen, um einen weiteren ml/min Clearance zu erzielen. Alternativ wird der Arbeitspunkt gesucht, bei dem eine weitere Erhöhung der Blutflussrate, die für die Erhöhung der Clearance um einen bestimmten Wert erforderlich wäre, einen bestimmten Wert nicht überschreitet.

Es können verschiedene Behandlungsmodi mit unterschiedlichen Effektivitäten der Blutbehandlung vorgegeben werden, wobei für den jeweiligen Behandlungsmodus diejenige Dialysierflüssigkeitsrate $Q_d$ oder Blutflussrate $Q_b$ ermittelt wird, bei deren Erhöhung um einen bestimmten Wert die Erhöhung der für die Effektivität der Blutbehandlung charakteristischen Größe eine dem jeweiligen Behandlungsmodus zugeordneten Wert nicht unterschreitet.

In der Praxis hat sich ein Verhältnis von 10:1 für den Quotienten $Q_d/K$ von Dialysierflüssigkeitsfluss $Q_d$ und Clearance K bewährt. Es ist aber grundsätzlich auch ein Verhältnis in einem Bereich von 5:1 bis 20:1 vertretbar.

Die optimale Dialysierflüssigkeitsrate $Q_{dopt}$ oder Blutflussrate $Q_{bopt}$ ist nicht nur von der Blutflussrate bzw. Dialysierflüssigkeitsrate, sondern auch von dem Dialysator abhängig, der für die Dialysebehandlung eingesetzt wird. Daher sehen die erfindungsgemäße Vorrichtung und das erfindungsgemäße Verfahren vor, die optimale Dialysierflüssigkeitsrate bzw. Blutflussrate in Abhängigkeit einer für den Dialysator charakteristischen Größe, insbesondere des Massentransferkoeffizienten k0A des Dialysators zu bestimmen.

Die optimale Dialysierflüssigkeitsflussrate $Q_{dopt}$ als Funktion des Blutflusses $Q_b$ und einer für den Dialysator charakteristischen Größe, insbesondere des Massentransferkoeffizienten k0A kann als dreidimensionales Kennlinienfeld in einem Speicher der Dialysevorrichtung abgelegt werden. Aus Speicherplatzgründen wird das Kennlinienfeld aber vorzugsweise durch eine geeignete mathematische Gleichung beschrieben, mit der für vorgegebene Blutflussraten und Massentransferkoeffizienten die optimale Dialysierflüssigkeitsrate berechnet werden kann. Das dreidimensionale Kennlinienfeld wird vorzugsweise durch ein Polynom höherer Ordnung, insbesondere ein Polynom dritter Ordnung in zwei oder mehreren Variablen inklusive aller Kreuzterme approximiert. Entsprechendes gilt für die Alternative eines optimalen Blutflusses als Funktion der Dialysierflüssigkeitsrate.

Die erfindungsgemäße Vorrichtung verfügt zur Berechnung der optimalen Dialysierflüssigkeitsrate bzw. Blutflussrate vorzugsweise über eine Recheneinheit, mit der für einen bestimmten Dialysator oder verschiedene Dialysatoren mit unterschiedlichen Massentransferkoeffizienten in Abhängigkeit von der Blutflussrate die optimale Dialysierflüssigkeitsrate oder umgekehrt berechnet wird.

Die erfindungsgemäße Vorrichtung sowie das erfindungsgemäße Verfahren zur Vorgabe einer Dialysierflüssigkeitsrate oder Blutflussrate können dazu Verwendung finden, dem behandelnden Arzt bei der Dialysebehandlung einen Vorschlag für die Einstellung einer optimalen Dialysierflüssigkeitsrate oder Blutflussrate zu geben. Die erfindungsgemäße Vorrichtung kann dabei Teil einer Blutbehandlungsvorrichtung sein oder eine separate Einheit bilden. Vorzugsweise verfügt aber die Blutbehandlungsvorrichtung bereits über die erfindungsgemäße Vorrichtung zur Vorgabe der optimalen Dialysierflüssigkeitsrate oder Blutflussrate. Weiterhin bevorzugt ist, dass die vorgegebene Dialysierflüssigkeitsrate oder Blutflussrate nicht dem behandelnden Arzt nur vorgeschlagen wird, sondern für die Blutbehandlung auch automatisch eingestellt wird.

Im Folgenden wird ein Ausführungsbeispiel der Erfindung unter Bezugnahme auf die Zeichnungen näher erläutert.

Es zeigen:

Figur 1    Die wesentlichen Komponenten einer erfindungsgemäßen Vorrichtung zur extrakorporalen Blutbehandlung mit einer erfindungsgemäßen Vorrichtung zur Vorgabe einer optimalen Dialysierflüssigkeitsrate in stark vereinfachter schematischer Darstellung.

Figur 2    die Clearance K(ml/min) als Funktion des Dialysierflüssigkeitsflusses $Q_d$ (ml/min) für einen bestimmten Dialysator für verschiedene Blutflusraten $Q_b$,

Figur 3    die zusätzliche Menge an Dialysierflüssigkeit, die bei verschiedenen Dialysierflüssigkeitsraten $Q_d$ und Blutflussraten $Q_b$ erforderlich ist, um die Clearance um 1 ml/min zu erhöhen.

Figur 4    ein dreidimensionales Kennlinienfeld, in dem die optimale Dialysierflüssigkeitsflussrate $Q_{dopt}$ als Funktion der Blutflussrate $Q_b$ und des Massentransferkoeffizienten des Dialysators dargestellt ist,

Figur 5    die Dialysierflüssigkeitsflussrate $Q_d$ (ml/min) als Funktion der Blutflussrate $Q_b$ (ml/min) bei dem erfindungsgemäßen Verfahren bzw. der erfindungsgemäßen Vorrichtung im Vergleich zu bekannten Verfahren,

Figur 6    die Clearance K (ml/min) als Funktion der Blutflussrate $Q_b$ (ml/min) bei dem erfindungsgemäßen Verfahren bzw. der erfindungsgemäßen Vorrichtung im Vergleich zu bekannten Verfahren,

Figur 7    die Differenz der Clearance $K_{opt}$ bei dem erfindungsgemäßen Verfahren bzw. der erfindungsgemäßen Vorrichtung und der Clearance K (A, B) bei bekannten Verfahren als Funktion der Blutflussrate $Q_b$ (ml/min),

Figur 8    der Quotient von optimaler Dialysierflüssigkeitsrate $Q_{dopt}$ und Blutflussrate $Q_b$ gemäß der Erfindung als Funktion der Blutflussrate $Q_b$ im Vergleich zu den bekannten Verfahren,

und Figur 9    der Quotient von optimaler Dialysierflüssigkeitsrate $Q_{dopt}$ und Blutflussrate $Q_b$ als Funktion der Blutflussrate $Q_b$ im Vergleich zu bekannten Verfahren.

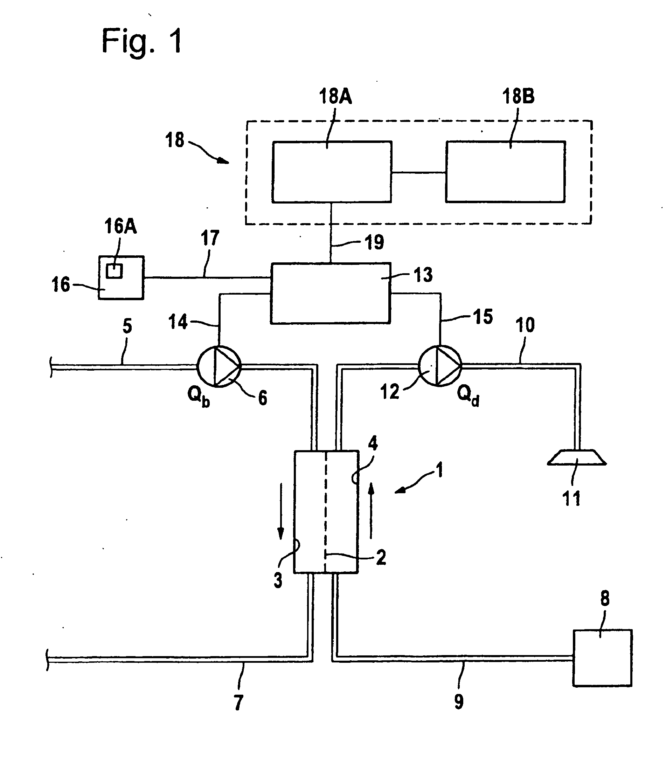

Figur 1 zeigt ein Ausführungsbeispiel einer erfindungsgemäßen Blutbehandlungsvorrichtung, die über eine erfindungsgemäße Vorrichtung zur Vorgabe einer optimalen Dialysierflüssigkeitsrate verfügt. Eine Blutbehandlungsvorrichtung, die über eine erfindungsgemäße Vorrichtung zur Vorgabe einer optimalen Blutflussrate verfügt, unterscheidet sich von der in Fig. 1 beschriebenen Vorrichtung nur dadurch, dass nach den erfindungsgemäßen Kriterien nicht in Abhängigkeit von der Blutflussrate $Q_b$ diejenige Dialysierflüssigkeitsrate $Q_d$, sondern in Abhängigkeit von der Dialysierflüssigkeitsrate $Q_d$ diejenige Blutflussrate $Q_b$ ermittelt wird, bei deren Erhöhung um einen bestimmten Wert die Erhöhung einer für die Effektivität der Blutbehandlung charakteristischen Größe einen bestimmten Wert nicht unterschreitet. Ansonsten umfassen beide Vorrichtungen die gleichen Komponenten.

In Fig. 1 sind der besseren Übersichtlichkeit halber nur die wesentlichen Komponenten der Blutbehandlungsvorrichtung dargestellt, da dem Fachmann die einzelnen Komponenten einer Blutbehandlungsvorrichtung zur Hämodialyse oder Hämodiafiltration allgemein bekannt sind.

Die erfindungsgemäße Dialysevorrichtung verfügt über einen Dialysator 1, der durch eine semipermeable Membran 2 in eine Blutkammer 3 und eine Dialysierflüssigkeitskammer 4 unterteilt ist. Von einem Patienten führt eine arterielle Blutleitung 5, in die eine Blutpumpe 6 geschaltet ist, zu einem Einlass der Blutkammer 3 des Dialysators, während von einem Auslass der Blutkammer eine venöse Blutleitung 7 zu dem Patienten führt.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu einem Einlass der Dialysierflüssigkeitskammer 4 des Dialysators 1, während eine Dialysierflüssigkeitsabführleitung 10 von einem Auslass der Dialysierflüssigkeitskammer zu einem Abfluss 11 führt. In die Dialysierflüssigkeitsabführleitung 10 ist eine Dialysierflüssigkeitspumpe 12 geschaltet.

Die Dialysevorrichtung verfügt über eine Steuereinheit 13, die mit der Blutpumpe 6 und der Dialysierflüssigkeitspumpe 12 über Steuerleitungen 14, 15 verbunden ist. Die Steuereinheit 13 erzeugt Steuersignale zum Betreiben der Blut- und Dialysierflüssigkeitspumpe 6,12 mit einer vorgegebenen Förderrate, so dass sich in der Blutleitung 5 eine vorgegebene Blutflussrate $Q_b$ und in der Dialysierflüssigkeitsleitung eine vorgegebene Dialysierflüssigkeitsrate $Q_d$ einstellen.

Zur Eingabe verschiedener Parameter für die Dialyse verfügt die Dialysevorrichtung über eine Eingabeeinheit 16, die beispielsweise eine alphanumerische Tastatur 16A aufweist. Mit der Eingabeeinheit 16 können neben verschiedenen anderen Größen beispielsweise die Blutflussrate $Q_b$ und eine für die Effektivität des verwendeten Dialysators 1 charakteristische Größe, insbesondere der Massentransferkoeffizient k0A des Dialysators eingegeben werden. Die Eingabeeinheit 16 ist über eine Datenleitung 17 mit der Steuereinheit 13 verbunden, mit der die einzelnen Komponenten der Dialysevorrichtung, insbesondere die Blut- und Dialysierflüssigkeitspumpe derart angesteuert werden, dass die Dialysebehandlung mit den vorgegebenen Dialyseparametern durchgeführt wird.

Die Dialysevorrichtung gibt für eine vorgegebene Blutflussrate $Q_b$ einen optimalen Dialysierflüssigkeitsfluss $Q_d$ vor. Hierzu verfügt die Dialysevorrichtung über eine Vorrichtung 18 zur Vorgabe des optimalen Dialysierflüssigkeitsflusses $Q_{dopt}$, deren Aufbau und Funktionsweise nachfolgend im Einzelnen beschrieben wird.

Es wird angenommen, dass die Dialysebehandlung mit einem bestimmten Dialysator 1 durchgeführt wird, der eine bestimmte Effektivität hat, die sich durch den Massentransferkoeffizient k0A des Dialysators angeben lässt. Für den Fall der Hämodialyse berechnet sich die Clearance K aus der Blutflussrate $Q_b$ und der Dialysierflüssigkeitsrate $Q_d$ und dem Massentransferkoeffizienten k0A des Dialysators 1 nach folgender Gleichung:

$$K = Q_b \frac{e^{k0A\left(\frac{1}{Q_b}-\frac{1}{Q_d}\right)} - 1}{e^{k0A\left(\frac{1}{Q_b}-\frac{1}{Q_d}\right)} - \frac{Q_b}{Q_d}}$$

$$(1)$$

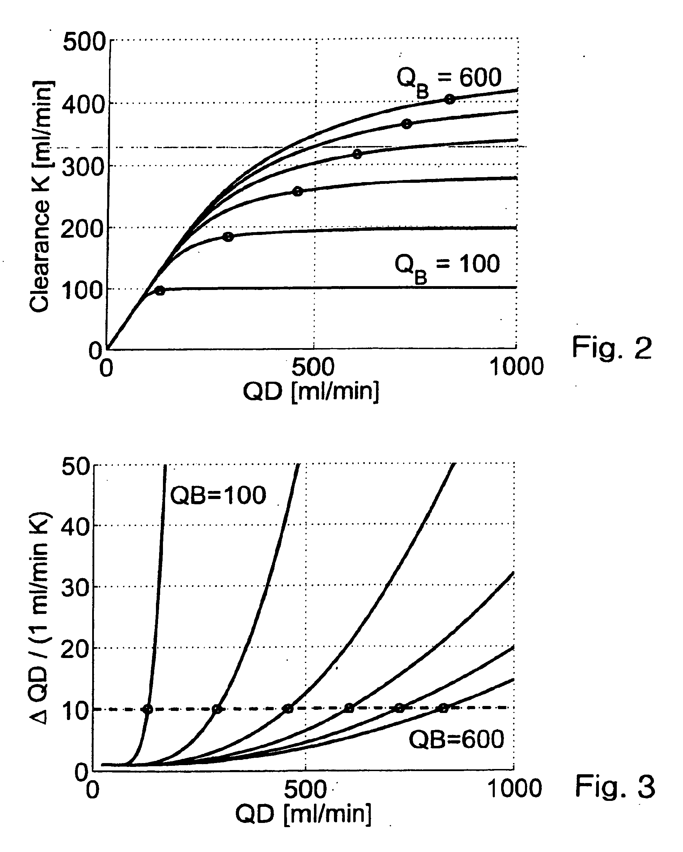

Figur 2 zeigt die Clearance K als Funktion der Dialysierflüssigkeitsrate $Q_d$ für verschiedene Blutflussraten $Q_b$. Es zeigt sich, dass bei hohen Dialysierflüssigkeitsraten $Q_d$ eine Sättigung der Clearance K eintritt. Daher ruft ab einer gewissen Blutflussrate eine Erhöhung der Dialysierflüssigkeitsrate keinen nennenswerten Gewinn an Clearance mehr hervor. Die erfindungsgemäße Vorrichtung gibt in Abhängigkeit von der Blutflussräte $Q_b$ eine optimale Dialysierflüssigkeitsrate $Q_{dopt}$ als optimalen Arbeitspunkt für die Dialysevorrichtung vor. Die optimalen Arbeitspunkte für unterschiedliche Blutflussraten sind in Figur 2 durch Kreise gekennzeichnet, wobei für die Arbeitspunkte ein Verhältnis von zusätzlicher Dialysierflüssigkeit pro ml/min zusätzlicher Clearance von 10:1 gewählt wurde. Wenn man ausgehend von dem jeweiligen Arbeitspunkt die Dialysierflüssigkeitsrate $Q_d$ weiter erhöht, so ist eine Erhöhung der Dialysierflüssigkeitsrate nicht mehr mit einer weiteren Erhöhung einer für die Dialysebehandlung charakteristischen Größe, insbesondere der Clearance K verbunden, die einen bestimmten Wert überschreitet. Die optimale Dialysierflüssigkeitsrate $Q_{dopt}$ ist also der Dialysierflüssigkeitsfluss, bei dessen Überschreiten die Ableitung der in Fig. 2 dargestellten Funktion, mit der die Abhängigkeit der für die Dialysebehandlung charakteristischen Größe, insbesondere der Clearance K, von der Dialysierflüssigkeitsrate $Q_d$ beschrieben wird, einen bestimmten Wert unterschreitet. Folglich wird nicht der absolute Wert der Clearance, sondern die Ableitung betrachtet.

Figur 3 zeigt, wie viele ml/min an zusätzlicher Dialysierflüssigkeit für unterschiedliche Blutflussraten $Q_b$ von 100 ml/min bis 600 ml/min erforderlich sind, um die Clearance K um einen ml/min zu erhöhen.

Versuche haben gezeigt, dass in der Praxis ein Verhältnis von 10:1 zu einem Arbeitspunkt führt, bei dem mit einem vertretbaren Verbrauch an Dialysierflüssigkeit eine relativ hohe Effektivität der Dialysebehandlung erzielt wird. Dieses Zielkriterium von 10:1 ist in Figur 3 durch eine horizontale gestrichelte Linie gekennzeichnet. Die jeweiligen Schnittpunkte mit den Grafen für die unterschiedlichen Blutflussraten stellen die verschiedenen Arbeitspunkte dar. Da sich die Ableitung der oben beschriebenen Funktion nach der Dialysierflüssigkeitsrate nicht ohne weiteres explizit berechnen lässt, sieht die Erfindung iterative Nährungslösungen vor.

In den Figuren 2 und 3 erkennt man deutlich, dass der Arbeitspunkt bei einer Blutflussrate $Q_b$ = 100 ml/min im Sättigungsbereich liegt. Im Arbeitspunkt bei $Q_b$ = 600 ml/min jedoch könnte man annehmen, dass eine weitere Erhöhung der Dialysierflüssigkeitsrate noch zu einem relevanten Gewinn an Clearance führt. Hierbei ist jedoch zu bedenken, dass die Dialysierflüssigkeitsrate rechts vom Arbeitspunkt um mehr als 10 ml/min erhöht werden müsste, um einen Gewinn an Clearance von nur einem ml/min zu erhalten.

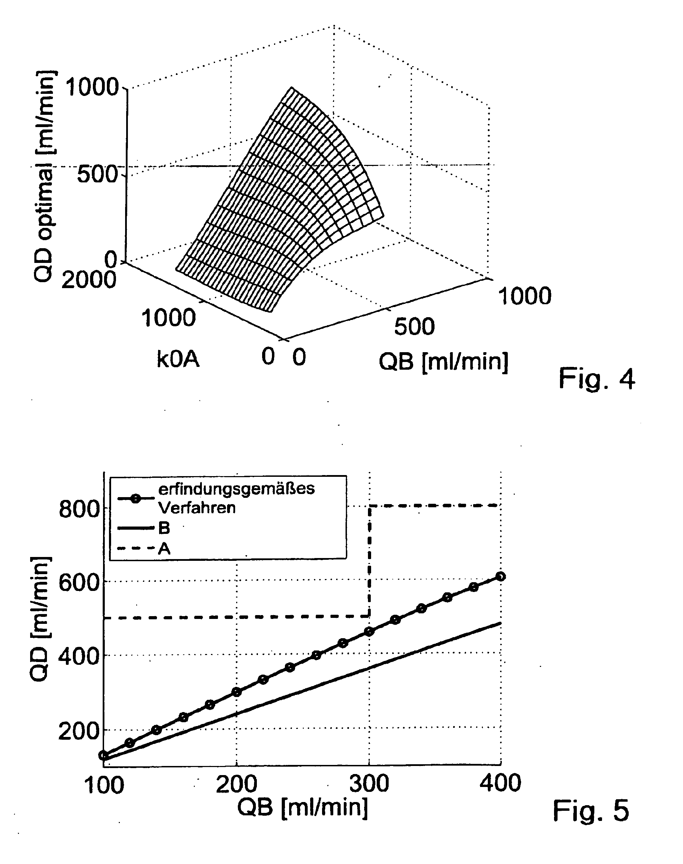

Die Figuren 2 und 3 zeigen die Clearance in Abhängigkeit vom Dialysierflüssigkeitsfluss nur für einen speziellen Dialysatortyp, der einen bestimmten Massentransferkoeffizienten k0A aufweist. In der Praxis können bei Dialysevorrichtungen aber unterschiedliche Dialysatoren Verwendung finden, die sich durch unterschiedliche Massentransferkoeffizienten unterscheiden. Figur 4 zeigt ein dreidimensionales Kennlinienfeld, mit dem sich in Abhängigkeit von der Blutflussrate $Q_b$ für unterschiedliche Dialysatoren, die sich jeweils durch einen bestimmten Massentransferkoeffizienten KOA auszeichnen, die optimale Dialysierflüssigkeitsflussrate $Q_{dopt}$ bestimmen lässt.

Das in Figur 4 dargestellte Kennlinienfeld könnte in Form einer Tabelle in einem Speicher der erfindungsgemäßen Vorrichtung 18 zur Vorgabe der optimalen Dialysierflüssigkeitsrate abgelegt sein. Aus Speicherplatzgründen sieht die Erfindung aber vor, das Kennlinienfeld durch eine geeignete Funktion zu approximieren. Hierzu sind verschiedene mathematische Verfahren bekannt.

Bei dem beschriebenen Ausführungsbeispiel wird das dreidimensionale Kennlinienfeld mit einem Polynom dritter Ordnung in beiden Achsen unter Verwendung aller möglichen Kreuzterme approximiert. Dies führt zu folgender Modell-Gleichung mit 4*4=16 Parametern a(i,j):

$$QD_{opt} = \alpha_{33} \cdot Q_b^3 k_{0A}^3 + \alpha_{32} \cdot Q_b^3 k_{0A}^2 + ... \alpha_{30} \cdot Q_b^3 k_{0A}^0$$

$$+ \alpha_{23} \cdot Q_b^2 k_{0A}^3 + \alpha_{22} \cdot Q_b^2 k_{0A}^2 + ... \alpha_{20} \cdot Q_b^2 k_{0A}^0$$

$$...$$

$$+ \alpha_{03} \cdot Q_b^0 k_{0A}^3 + \alpha_{02} \cdot Q_b^0 k_{0A}^2 + ... \alpha_{00} \qquad (2)$$

Die einzelnen Parameter des obigen Gleichungssystems werden nach dem Least-Squares-Verfahren bestimmt, so dass die Summe der quadratischen Abweichungen zwischen Rohdaten und Modell minimiert wird. Die Übereinstimmung des Kennfeldes (Fläche) und der Modell-Gleichung ist in der Praxis ausreichend.

Für die Durchführung der Dialysebehandlung gibt der behandelnde Arzt eine bestimmte Blutflussrate $Q_b$ vor, die er mit der Tastatur 16 A der Eingabeeinheit 16 eingibt, woraufhin die Steuereinheit 13 die Förderrate (Drehzahl) der Blutpumpe 6 entsprechend einstellt. Weiterhin gibt der Arzt mit der Eingabeeinheit 16 ein, welcher Dialysator 1 für die Dialysebehandlung eingesetzt wird, woraufhin der zu dem jeweiligen Dialysatortyp gehörige Massentransferkoeffizient k0A bestimmt wird, der in einem Speicher abgelegt ist. Es ist aber auch möglich, den jeweiligen Massentransferkoeffizienten k0A des verwendeten Dialysators direkt einzugeben.

Die Werte für die vorgegebene Blutflussrate $Q_b$ und den vorgegebenen Massentransferkoeffizienten k0A erhält die Vorrichtung 18 über die Datenleitung 19 von der Steuereinheit 13. Die Vorrichtung 18 verfügt über eine Recheneinheit 18A, die auf der Grundlage der oben beschriebenen Gleichung dritter Ordnung den optimalen Dialysierflüssigkeitsfluss $Q_{dopt}$ berechnet. Zur Anzeige des optimalen Dialysierflüssigkeitsflusses $Q_{dopt}$ verfügt die Vorrichtung 18 über eine Anzeigeeinheit 18B, beispielsweise in Form eines Bildschirms oder eines Displays.

Darüber hinaus gibt die Vorrichtung 18 den berechneten Wert für den optimalen Dialysierflüssigkeitsfluss $Q_{dopt}$ über die Datenleitung 19 an die Steuereinheit 16 aus, die wiederum die Drehzahl der Dialysierflüssigkeitspumpe 12 derart einstellt, dass die Dialysierflüssigkeit mit der optimalen Dialysierflüssigkeitsrate $Q_{dopt}$ gefördert wird.

In einer bevorzugten Ausführungsform sieht die Eingabeeinheit 16 eine Eingabe unterschiedlicher Zielkriterien vor. Beispielsweise kann als Zielkriterium neben dem oben beschriebenen Verhältnis von 10:1 ein Verhältnis von 5:1, d.h. 5 ml/min an zusätzlicher Dialysierflüssigkeit für ein ml/min an zusätzlicher Clearance sowie ein Verhältnis von 15: 1 oder 20:1 eingestellt werden.

In Abhängigkeit von dem eingestellten Modus (5:1, 10:1 sowie 15:1 oder 20:1) berechnet die Recheneinheit 18A der Vorrichtung 18 dann den optimalen Dialysierflüssigkeitsfluss $Q_{dopt}$. Dabei entspricht das Verhältnis 5:1 einem wirtschaftlichen Modus, in dem zwar Dialysierflüssigkeit gespart werden soll, aber nicht die gewohnte Clearance erreicht werden kann, das Verhältnis 10:1 einem Normal-Modus und das Verhältnis 15:1 oder 20:1 einem Intensiv-Modus, mit dem eine besonders hohe Clearance aber unter Verwendung von einer größeren Menge an Dialysierflüssigkeit erzielt werden soll.

Für den Fall, dass bei der Hämodialyse auch der Ultrafiltratfluss Berücksichtigung findet, ergibt sich anstelle der oben genannten Gleichung (1) die folgende Gleichung (1') für die Hämodialyse unter Berücksichtigung der Ultrafiltration:

$$K = \frac{1 - \exp\left[\frac{k_0 A}{Q_B} \cdot \left(1 - \frac{Q_B}{Q_D}\right)\right]}{\frac{Q_B}{Q_D} - \exp\left[\frac{k_0 A}{Q_B} \cdot \left(1 - \frac{Q_B}{Q_D}\right)\right]} \cdot Q_B \cdot \left(1 - \frac{Q_F}{Q_B}\right) + Q_F$$

Gleichung (1')

mit

$Q_B$    Blutfluß
$Q_D$    Dialysatfluß
$Q_F$    Filtratfluß, hier nur Ultrafiltration
$k_0A$    Massentransferflächenkoeffizient

Nachfolgend wird der allgemeinere Fall der Hämodiafiltration beschrieben, bei der neben der Hämodialyse auch eine Hämofiltration erfolgt. Für den Fall der Hämodiafiltration wird der Zusammenhang der Flussraten durch die folgende allgemeinere Gleichung (1") für die Hämodiafiltration beschrieben.

$$K = Q_{Bi} \cdot \frac{1 - \dfrac{Q_{Do}}{Q_{Di}} \cdot \dfrac{Q_{Bo}}{Q_{Bi}} Z}{1 - \dfrac{Q_{Bo}}{Q_{Di}} Z} \qquad\qquad \text{Gleichung (1'')}$$

mit

$$Z = \left(1 - \frac{Q_F}{Q_{Bi}}\right)^{\frac{p}{Q_F} - 1} \cdot \left(1 - \frac{Q_F}{Q_{Do}}\right)^{-\left(\frac{p}{Q_F} - 1\right)}$$

$$p = k_0 A + (1 - \sigma) \cdot (1 - f) \cdot Q_F$$

$$f = \frac{1}{Pe} - \frac{1}{\exp(Pe) - 1}$$

$$Pe = (1 - \sigma) \cdot \frac{Q_F}{k_0 A}$$

mit

$\sigma$    Reflexionskoeffizient (0< $\sigma$ <1), z.B. für Harnstoff $\sigma$ =0
i    Index für Zufluß (inflow)
o    Index für Abfluß (outflow)
$Q_F$    Gesamte Filtrationsrate, Summe aus Ultrafiltrationsrate $Q_{UF}$ und Substitutionsrate Qs

$$Q_F = Q_{UF} + Q_S$$

$Q_{Di}$    Fluß, der am Dialysateingang des Dialysators anliegt, entspricht i.d.R. dem an der Maschine eingestellten Dialysatfluß $Q_D$
$Q_{Do}$    Fluß, der am Dialysatausgang des Dialysators anliegt

$$Q_{Do} = Q_{Di} + Q_F$$

$Q_{Bi}$ Fluß, der am Bluteingang des Dialysators anliegt

$$Q_{Bi} = Q_B + f \cdot Q_S$$

mit f Faktor für Prä bzw. Postdilution, f=0 bei Postdilution, f=1 für Prädilution, bzw. zwischen 0 und 1 bei mixed-dilution (gemischter Verdünnung)

$Q_{Bo}$ Fluß, der am Blutausgang des Dialysators anliegt

$$Q_{Bo} = Q_{Bi} - Q_F$$

$Q_B$ Blutfluß, der im extrakorpralen Kreislauf vor der arteriellen.Infusionsstelle anliegt, entspricht dem vom Anwender eingestellten Blutfluß

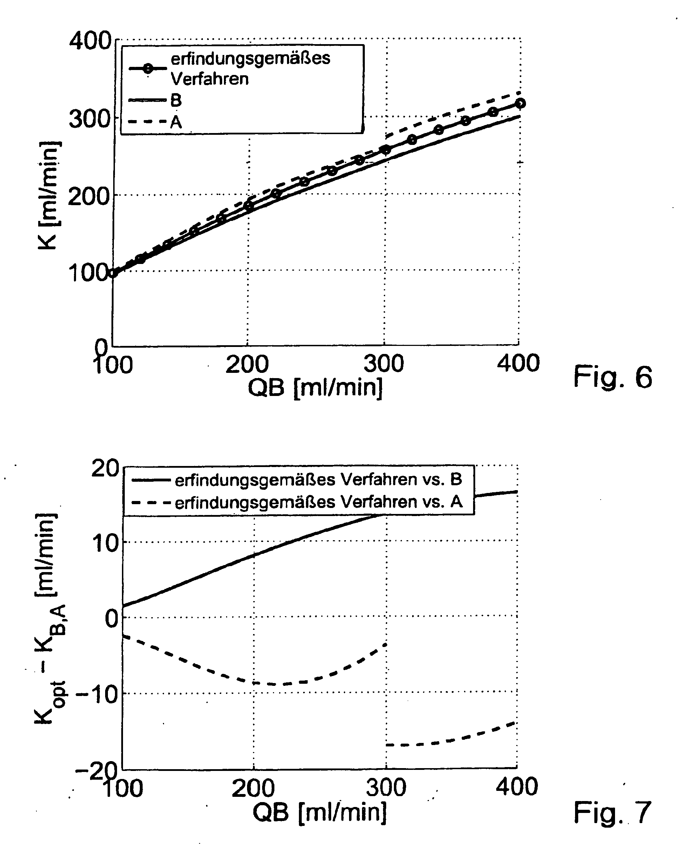

Nachfolgend werden die Unterschiede des erfindungsgemäßen Verfahrens im Vergleich zu den bekannten Verfahren zur Vorgabe einer bestimmten Dialysierflüssigkeitsrate unter Bezugnahme auf die Figuren 5 bis 9 beschrieben. In den Figuren 5 bis 9 ist ein bekanntes Verfahren, bei dem eine konstante Dialysierflüssigkeitsrate von 500 ml/min für Blutflussraten $Q_b$ bis zu 300 ml/min und eine konstante Dialysierflüssigkeitsrate $Q_d$ von 800 ml/min für Blutflussraten $Q_b$ größer als 300 ml/min eingestellt werden mit "A" bezeichnet. Ein bekanntes Verfahren, bei dem die Dialysierflüssigkeitsrate durch Multiplikation der Blutflussrate $Q_b$ mit einem Faktor von 1,2 berechnet wird, ist mit "B" gekennzeichnet. Figur 5 zeigt die Dialysierflüssigkeitsrate Qd als Funktion der Blutflussrate $Q_b$ für das erfindungsgemäße Verfahren im Vergleich zu den bekannten Verfahren "A" und "B", während Figur 6 die Clearance K als Funktion des Blutflusses für das erfindungsgemäße Verfahren und die bekannten Verfahren "A" und "B" zeigt.

Figur 7 zeigt die Differenz zwischen der mit dem erfindungsgemäßen Verfahren bei der optimalen Dialysierflüssigkeitsrate $Q_{dopt}$ erzielten Clearance $K_{opt}$ und der mit den bekannten Verfahren erzielten Clearance $K_A$ bzw. $K_B$ als Funktion der Blutflussrate $Q_b$, um die Unterschiede zwischen dem erfindungsgemäßen Verfahren und den bekannten Verfahren im Hinblick auf die Veränderung der Clearance deutlicher werden zu lassen. Gegenüber dem bekannten Verfahren "A" wird mit dem erfindungsgemäßen Verfahren eine relativ große Menge an Dialysierflüssigkeit gespart, obwohl die Clearance nur um einen relativ kleinen Betrag verringert ist. Zwar wird mit dem Verfahren "B" gegenüber dem Verfahren A noch mehr Dialysierflüssigkeit gespart, es führt aber auch zu einer größeren Reduzierung der Clearance.

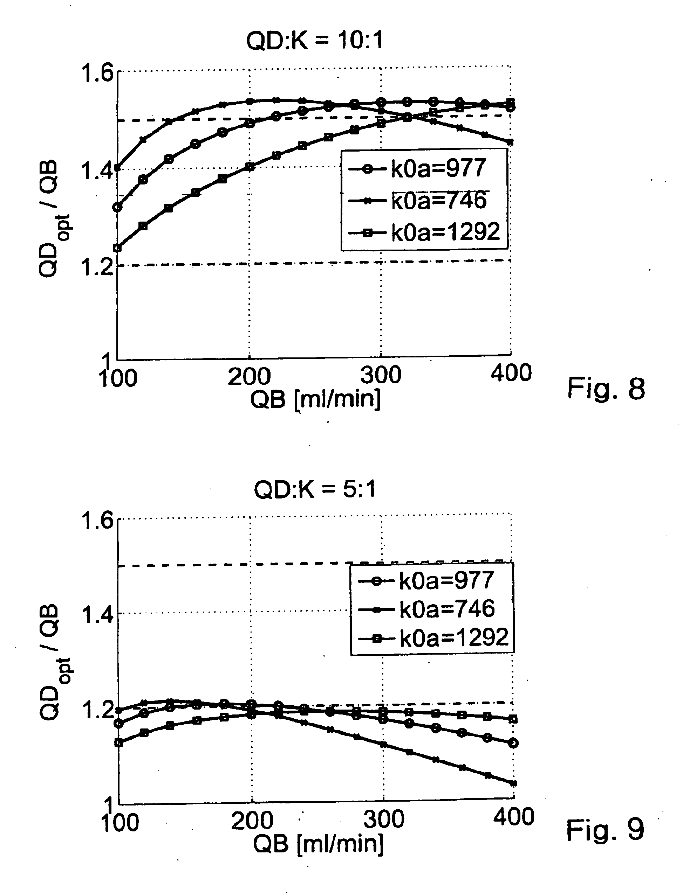

Die Figuren 8 und 9 zeigen für die gewählten "Zielkriterien" von 10:1 (Figur 8) und 5:1 (Figur 9) die auf die Blutflussrate normierte optimale Dialysierflüssigkeitsflussrate $Q_{dopt}$ nach dem erfindungsgemäßen Verfahren für drei verschiedene Dialysatoren. Man erkennt, dass für das gewählte Zielkriterium von 10:1 ein Faktor von 1,5 im Arbeitsbereich zwischen $Q_b$ = 200 und 400 nur als erste Nährung für verschiedene Dialysatoren plausibel ist. Der Faktor 1,2 liegt hingegen für das 10:1-Zielkriterium zu tief. Die Sachlage ändert sich jedoch, wenn man ein Zielkriterium von 5:1, d.h. 5 ml/min Dialysierflüssigkeit für 1 ml/min Clearance wählt. Hier erkennt man, dass ein Faktor von 1,2 eine bessere Näherung darstellt. Mit der erfindungsgemäßen Lehre gelingt es, einen Kompromiss zwischen den Verfahren A und B zu finden, wobei dem Anwender gleichzeitig quantifizierte Arbeitspunkte an die Hand gegeben werden, bei denen er die Auswirkungen von Flusserhöhungen auf die Effektivität der Behandlung unmittelbar überblick.

**Patentansprüche**

1. Vorrichtung zur Vorgabe einer Dialysierflüssigkeitsrate oder einer Blutflussrate für eine extrakorporale Blutbehandlungvorrichtung, die einen Dialysator aufweist, der durch eine semipermeable Membran in eine Blutkammer, die von Blut mit einer vorgegebenen Blutflussrate $Q_b$ durchströmt wird, und eine Dialysierflüssigkeitskammer unterteilt ist, die von Dialysierflüssigkeit mit einer vorgegebenen Dialysierflüssigkeitsrate $Q_d$ durchströmt wird, wobei die Vorrichtung zur Vorgabe der Dialysierflüssigkeitsrate oder der Blutflussrate die Dialysierflüssigkeitsrate $Q_d$ oder Blutflussrate $Q_b$ in Abhängigkeit von der vorgegebenen Blutflussrate $Q_b$ bzw. Dialysierflüssigkeitsrate $Q_d$ ermittelt,

**dadurch gekennzeichnet, dass**

die Vorrichtung zur Vorgabe der Dialysierflüssigkeitsrate derart ausgebildet ist, dass bei einer vorgegebenen Blutflussrate $Q_b$ diejenige Dialysierflüssigkeitsrate $Q_d$ oder bei einer vorgegebenen Dialysierflüssigkeitsrate $Q_d$ diejenige Blutflussrate $Q_b$ ermittelt wird, bei deren Erhöhung um einen bestimmten Wert die Erhöhung einer für die Effektivität der Blutbehandlung charakteristischen Größe einen bestimmten Wert nicht unterschreitet.

2. Vorrichtung nach Anspruch 1, **dadurch gekennzeichnet, dass** die für die Dialysebehandlung charakteristische Größe die Clearance K der Dialysebehandlung ist.

3. Vorrichtung nach Anspruch 1 oder 2, **dadurch gekennzeichnet, dass** die Vorrichtung zur Vorgabe der Dialysierflüssigkeitsrate oder Blutflussrate derart ausgebildet ist, dass diejenige Dialysierflüssigkeitsrate $Q_d$ bzw. Blutflussrate $Q_b$, bei deren Erhöhung um einen bestimmten Wert die Erhöhung einer für die Effektivität der Blutbehandlung charakteristischen Größe einen bestimmten Wert nicht unterschreitet, in Abhängigkeit von einer für den Dialysator charakteristischen Größe ermittelt wird.

4. Vorrichtung nach Anspruch 3, **dadurch gekennzeichnet, dass** die für den Dialysator charakteristische Größe der Massentransferkoeffizient K0A des Dialysators ist.

5. Vorrichtung nach einem der Ansprüche 1 bis 4, **dadurch gekennzeichnet, dass** die Vorrichtung zur Vorgabe der Dialysierflüssigkeitsrate oder Blutflussrate eine Eingabeeinheit (16) zur Vorgabe verschiedener Behandlungsmodi mit unterschiedlichen Effektivitäten der Blutbehandlung aufweist, wobei für den jeweiligen Behandlungsmodus diejenige Dialysierflüssigkeitsrate $Q_d$ bzw. Blutflussrate $Q_b$ ermittelt wird, bei deren Erhöhung um einen bestimmten Wert die Erhöhung der für die Effektivität der Blutbehandlung charakteristischen Größe eine dem jeweiligen Behandlungsmodus zugeordneten Wert nicht unterschreitet.

6. Vorrichtung nach einem der Ansprüche 1 bis 5, **dadurch gekennzeichnet, dass** die Vorrichtung zur Vorgabe der Dialysierflüssigkeitsrate oder Blutflussrate eine Recheneinheit (18A) aufweist, die derart ausgebildet ist, dass die Diaysierflüssigkeitsrate $Q_d$ bzw. Blutflussrate $Q_b$ nach einer mathematischen Gleichung berechnet wird.

7. Vorrichtung nach Anspruch 6, **dadurch gekennzeichnet, dass** die mathematische Gleichung zum Berechnen der Dialysierflüssigkeitsrate $Q_d$ ein Polynom mehrfacher Ordnung ist.

8. Blutbehandlungsvorrichtung für eine extrakorporale Blutbehandlung mit einem Dialysator (1), der durch eine semipermeable Membran (2) in eine Blutkammer (3), die von Blut mit einer vorgegebenen Blutflussrate $Q_b$ durchströmt wird, und eine Dialysierflüssigkeitskammer (4) unterteilt ist, die von Dialysierflüssigkeit mit einer vorgegebenen Dialysierflüssigkeitsrate $Q_d$ durchströmt wird, **dadurch gekennzeichnet, dass** die Blutbehandlungsvorrichtung eine Vorrichtung (18) zur Vorgabe einer Dialysierflüssigkeitsrate oder Blutflussrate nach einem der Ansprüche 1 bis 7 aufweist.

9. Blutbehandlungsvorrichtung nach Anspruch 8, **dadurch gekennzeichnet, dass** die Blutbehandlungsvorrichtung eine Steuereinheit (13) aufweist, mit der die von der Vorrichtung zur Vorgabe der Dialysierflüssigkeitsrate oder Blutflussrate vorgegebene Dialysierflüssigkeitsrate $Q_d$ oder Blutflussrate $Q_b$ eingestellt wird.

10. Blutbehandlungsvorrichtung nach Anspruch 9, **dadurch gekennzeichnet, dass** die Blutbehandlungsvorrichtung eine Dialysierflüssigkeitspumpe (12) zum Fördern der Dialysierflüssigkeit mit einer bestimmten Dialysierflüssigkeitsrate $Q_d$ und/oder eine Blutpumpe (6) zum Fördern von Blut mit einer bestimmten Blutflussrate $Q_b$ aufweist, wobei die Steuereinheit (13) die Drehzahl der Dialysierflüssigkeitspumpe bzw. die Drehzahl der Blutpumpe derart einstellt, dass die Dialysierflüssigkeit bzw. das Blut mit der von der Vorrichtung zur Vorgabe der Dialysierflüssigkeitsrate oder Blutflussrate vorgegebenen Dialysierflüssigkeitsrate $Q_d$ bzw. Blutflussrate $Q_b$ gefördert wird.

11. Verfahren zur Vorgabe einer Dialysierflüssigkeitsrate oder Blutflussrate für eine extrakorporale Blutbehandlungvorrichtung, die einen Dialysator aufweist, der durch eine semipermeable Membran in eine Blutkammer, die von Blut mit einer vorgegebenen Blutflussrate $Q_b$ durchströmt wird, und eine Dialysierflüssigkeitskammer unterteilt ist, die von Dialysierflüssigkeit mit einer vorgegebenen Dialysierflüssigkeitsrate $Q_d$ durchströmt wird, wobei die Dialysierflüssigkeitsrate $Q_d$ oder Blutflussrate $Q_b$ in Abhängigkeit von der vorgegebenen Blutflussrate $Q_b$ bzw. Dialysierflüssigkeitsrate $Q_d$ ermittelt wird, **dadurch gekennzeichnet, dass** in Abhängigkeit von der Blutflussrate $Q_b$ diejenige Dialysierflüssigkeitsrate $Q_d$ oder in Abhängigkeit von der Dialysierflüssigkeitsrate $Q_d$ diejenige Blutflussrate $Q_b$ ermittelt wird, bei deren Erhöhung um einen bestimmten Wert die Erhöhung einer für die Effektivität der Blutbehandlung charakteristischen Größe einen bestimmten Wert nicht unterschreitet.

12. Verfahren nach Anspruch 11, **dadurch gekennzeichnet, dass** die für die Dialysebehandlung charakteristische Größe die Clearance K der Dialysebehandlung ist.

13. Verfahren nach Anspruch 11 oder 12, **dadurch gekennzeichnet, dass** diejenige Dialysierflüssigkeitsrate $Q_d$ oder Blutflussrate $Q_b$, bei deren Erhöhung um einen bestimmten Wert die Erhöhung einer für die Effektivität der Blutbehandlung charakteristischen Größe einen bestimmten Wert nicht unterschreitet, in Abhängigkeit von einer für den Dialysator charakteristischen Größe ermittelt wird.

14. Verfahren nach Anspruch 13, **dadurch gekennzeichnet, dass** die für den Dialysator charakteristische Größe der Massentransferkoeffizient K0A des Dialysators ist.

15. Verfahren nach einem der Ansprüche 11 bis 14, **dadurch gekennzeichnet, dass** verschiedene Behandlungsmodi mit unterschiedlichen Effektivitäten der Blutbehandlung vorgegeben werden, wobei für den jeweiligen Behandlungsmodus diejenige Dialysierflüssigkeitsrate $Q_d$ oder Blutflussrate $Q_b$ ermittelt wird, bei deren Erhöhung um einen bestimmten Wert die Erhöhung der für die Effektivität der Blutbehandlung charakteristischen Größe eine dem jeweiligen Behandlungsmodus zugeordneten Wert nicht unterschreitet.

16. Verfahren nach einem der Ansprüche 11 bis 15, **dadurch gekennzeichnet, dass** die Diaysierflüssigkeitsrate $Q_d$ bzw. Blutflussrate $Q_b$ nach einer mathematischen Gleichung berechnet wird.

17. Verfahren nach Anspruch 16, **dadurch gekennzeichnet, dass** die mathematische Gleichung zum Berechnen der Dialysierflüssigkeitsrate $Q_d$ ein Polynom mehrfacher Ordnung ist.

**Claims**

1. Apparatus for setting a dialysis fluid rate or a blood flow rate for an extracorporeal blood treatment apparatus, which has a dialyzer that is divided, by means of a semi-permeable membrane, into a blood chamber through which blood flows at a set blood flow rate $Q_b$ and a dialysis fluid chamber through which dialysis fluid flows at a set dialysis fluid rate $Q_d$,
wherein the apparatus for setting the dialysis fluid rate or the blood flow rate determines the dialysis fluid rate $Q_d$ or blood flow rate $Q_b$ as a function of the set blood flow rate $Q_b$ or dialysis fluid rate $Q_d$, respectively,
**characterized in that**
the apparatus for setting the dialysis fluid rate is configured in such a manner that at a set blood flow rate $Q_b$, the particular dialysis fluid rate $Q_d$ is determined, or at a set dialysis fluid rate $Q_d$, the particular blood flow rate $Q_b$ is determined, when if this particular rate is increased by a specific value, the increase in a variable that is characteristic for the efficacy of the blood treatment is not less than a specific value.

2. Apparatus according to claim 1, **characterized in that** the variable that is characteristic for the dialysis treatment is the clearance K of the dialysis treatment.

3. Apparatus according to claim 1 or 2, **characterized in that** the apparatus for setting the dialysis fluid rate or blood flow rate is configured in such a manner that the particular dialysis fluid rate $Q_d$ or blood flow rate $Q_b$, which, when it is increased by a specific value, the increase in a variable that is characteristic for the efficacy of the blood treatment is not less than a specific value, is determined as a function of a variable that is characteristic for the dialyzer.

4. Apparatus according to claim 3, **characterized in that** the variable that is characteristic for the dialyzer is the mass transfer coefficient KOA of the dialyzer.

5. Apparatus according to one of claims 1 to 4, **characterized in that** the apparatus for setting the dialysis fluid rate or blood flow rate has an input unit (16) for setting different treatment modes with different efficacies of the blood treatment, wherein for the treatment mode, in each instance, the particular dialysis fluid rate $Q_d$ or blood flow rate $Q_b$ is determined, when if this particular rate is increased by a specific value, the increase in a variable that is characteristic for the efficacy of the blood treatment is not less than the value assigned to the treatment mode, in each instance.

6. Apparatus according to one of claims 1 to 5, **characterized in that** the apparatus for setting the dialysis fluid rate or blood flow rate has a calculation unit (18A) that is configured in such a manner that the dialysis fluid rate $Q_d$ or the blood flow rate $Q_b$, respectively, is calculated according to a mathematical equation.

7. Apparatus according to claim 6, **characterized in that** the mathematical equation for calculating the dialysis fluid rate $Q_d$ is a polynomial of a multiple order.

8. Blood treatment apparatus for extracorporeal blood treatment with a dialyzer (1) that is divided, by means of a semi-permeable membrane (2), into a blood chamber (3) through which blood flows at a set blood flow rate $Q_b$ and a dialysis fluid chamber (4) through which dialysis fluid flows at a set dialysis fluid rate $Q_d$, **characterized in that** the blood treatment apparatus has an apparatus (18) for setting a dialysis fluid rate or blood flow rate according to one of claims 1 to 7.

9. Blood treatment apparatus according to claim 8, **characterized in that** the blood treatment apparatus has a control unit (13) with which the dialysis fluid rate $Q_d$ or blood flow rate $Q_b$ set by the apparatus for setting the dialysis fluid rate or blood flow rate is set.

10. Blood treatment apparatus according to claim 9, **characterized in that** the blood treatment apparatus has a dialysis fluid pump (12) for conveying the dialysis fluid at a specific dialysis fluid rate $Q_d$ and/or a blood pump (6) for conveying blood at a specific blood flow rate $Q_b$, wherein the control unit (13) sets the speed of rotation of the dialysis fluid pump or the speed of rotation of the blood pump in such a manner that the dialysis fluid or the blood is conveyed at the dialysis fluid rate $Q_d$ or blood flow rate $Q_b$ set by the apparatus for setting the dialysis fluid rate or blood flow rate.

11. Method for setting a dialysis fluid rate or blood flow rate for an extracorporeal blood treatment apparatus, which has a dialyzer that is divided, by means of a semi-permeable membrane, into a blood chamber through which blood flows at a set blood flow rate $Q_b$ and a dialysis fluid chamber through which dialysis fluid flows at a set dialysis fluid rate $Q_d$,
wherein the dialysis fluid rate $Q_d$ or blood flow rate $Q_b$ is determined as a function of the set blood flow rate $Q_b$ or dialysis fluid rate $Q_d$, respectively,
**characterized in that**
as a function of the blood flow rate $Q_b$, the particular dialysis fluid rate $Q_d$ is determined, or as a function of the dialysis fluid rate $Q_d$, the particular blood flow rate $Q_b$ is determined, when if this particular rate is increased by a specific value, the increase in a variable that is characteristic for the efficacy of the blood treatment is not less than a specific value.

12. Method according to claim 11, **characterized in that** the variable that is characteristic for the dialysis treatment is the clearance K of the dialysis treatment.

13. Method according to claim 11 or 12, **characterized in that** the particular dialysis fluid rate $Q_d$ or blood flow rate $Q_b$, which, when if this particular rate is increased by a specific value, the increase in a variable that is characteristic for the efficacy of the blood treatment is not less than a specific value, is determined as a function of a variable that is characteristic for the dialyzer.

14. Method according to claim 13, **characterized in that** the variable that is characteristic for the dialyzer is the mass transfer coefficient KOA of the dialyzer.

15. Method according to one of claims 11 to 14, **characterized in that** different treatment modes having different efficacies of the blood treatment are set, wherein the particular dialysis fluid rate $Q_d$ or blood flow rate $Q_b$ is determined for the treatment mode, in each instance, when if this particular rate is increased by a specific value, the increase in the variable that is characteristic for the efficacy of the blood treatment is not less than a specific value assigned to the treatment mode, in each instance.

16. Method according to one of claims 11 to 15, **characterized in that** the dialysis fluid rate $Q_d$ or blood flow rate $Q_b$ is calculated according to a mathematical equation.

17. Method according to claim 16, **characterized in that** the mathematical equation for calculating the dialysis fluid rate $Q_d$ is a polynomial of a multiple order.

**Revendications**

1. Dispositif de définition d'un débit de liquide de dialyse ou d'un débit sanguin pour un dispositif de traitement sanguin extracorporel qui présente un dialyseur qui est subdivisé par une membrane semi-perméable en une chambre de sang, qui est traversée par le sang à un débit sanguin $Q_b$ prédéterminé, et en une chambre de liquide de dialyse, qui est traversée par un liquide de dialyse à un débit de liquide de dialyse $Q_d$ prédéterminé,
Dans lequel le dispositif de définition du débit du liquide de dialyse ou du débit sanguin détermine le débit du liquide de dialyse $Q_d$ ou le débit sanguin $Q_b$ en fonction du débit sanguin $Q_b$ ou du débit de liquide de dialyse $Q_d$ prédéterminés,
**caractérisé en ce que**
le dispositif de définition du débit du liquide de dialyse est conçu de telle sorte que pour un débit sanguin prédéterminé $Q_b$ le débit du liquide de dialyse $Q_d$ ou pour un débit du liquide de dialyse prédéterminé $Q_d$ le débit sanguin $Q_b$ est déterminé, dans le cas de leur augmentation d'une valeur déterminée, l'augmentation d'une grandeur caractéristique pour l'efficacité du traitement sanguin ne dépassant pas une valeur déterminée.

2. Dispositif selon la revendication 1, **caractérisé en ce que** la grandeur caractéristique pour le traitement de dialyse est la clairance K du traitement de dialyse.

3. Dispositif selon la revendication 1 ou 2, **caractérisé en ce que** le dispositif de définition du débit du liquide de dialyse ou du débit sanguin est conçu de telle sorte que le débit du liquide de dialyse $Q_d$ ou le débit sanguin $Q_b$, dans le cas de leur augmentation d'une valeur déterminée, ne dépasse pas l'augmentation d'une grandeur caractéristique pour l'efficacité du traitement sanguin d'une valeur déterminée, en fonction de la grandeur caractéristique pour le dialyseur.

4. Dispositif selon la revendication 3, **caractérisé en ce que** la grandeur caractéristique pour le dialyseur est le coefficient de transfert de masse KOA du dialyseur.

5. Dispositif selon l'une des revendications 1 à 4, **caractérisé en ce que** le dispositif de définition du débit du liquide de dialyse ou du débit sanguin présente une unité d'entrée (16) pour la définition de divers modes de traitement ayant différentes efficacités du traitement sanguin, dans lequel, pour le mode de traitement respectif, le débit du liquide de dialyse $Q_d$ ou le débit sanguin $Q_b$ est déterminé, dans le cas de leur augmentation d'une valeur déterminée, l'augmentation de la grandeur caractéristique pour l'efficacité du traitement sanguin ne dépassant pas une valeur attribuée au mode de traitement respectif.

6. Dispositif selon l'une des revendications 1 à 5, **caractérisé en ce que** le dispositif de définition du débit du liquide de dialyse ou du débit sanguin présente une unité de calcul (18A) qui est conçue de telle sorte que le débit du liquide de dialyse $Q_d$ ou le débit sanguin $Q_b$ est calculé selon une équation mathématique.

7. Dispositif selon la revendication 6, **caractérisé en ce que** l'équation mathématique pour le calcul du débit du liquide de dialyse $Q_d$ est un polynôme de degrés multiples.

8. Dispositif de traitement sanguin pour le traitement sanguin extracorporel avec un dialyseur (1) qui est subdivisé par une membrane semi-perméable (2) en une chambre de sang (3), qui est traversée par le sang à un débit sanguin prédéterminé $Q_b$, et en une chambre de liquide de dialyse (4), qui est traversée par un liquide de dialyse à un débit de liquide de dialyse $Q_d$ prédéterminé, **caractérisé en ce que** le dispositif de traitement sanguin présente un dispositif (18) de définition d'un débit du liquide de dialyse ou d'un débit sanguin selon l'une des revendications 1 à 7.

9. Dispositif de traitement sanguin selon la revendication 8, **caractérisé en ce que** le dispositif de traitement sanguin présente une unité de commande (13) avec laquelle le débit du liquide de dialyse $Q_d$ ou le débit sanguin $Q_b$ prédéterminé par le dispositif de définition du débit du liquide de dialyse ou du débit sanguin est réglé.

10. Dispositif de traitement sanguin selon la revendication 9, **caractérisé en ce que** le dispositif de traitement sanguin présente une pompe de liquide de dialyse (12) pour acheminer le liquide de dialyse à un débit du liquide de dialyse $Q_d$ prédéterminé et/ou une pompe de sang (6) pour acheminer le sang à un débit sanguin $Q_b$ prédéterminé, dans lequel l'unité de commande (13) ajuste le nombre de tours de la pompe de liquide de dialyse ou le nombre de tours de la pompe de sang de telle sorte que le liquide de dialyse ou le sang soit acheminé au débit du liquide de dialyse $Q_d$ ou au débit sanguin $Q_b$ prédéterminé par le dispositif de définition du débit du liquide de dialyse ou du débit sanguin.

**11.** Procédé de définition d'un débit du liquide de dialyse ou d'un débit sanguin pour un dispositif de traitement sanguin extracorporel qui présente un dialyseur qui est subdivisé par une membrane semi-perméable en une chambre de sang, qui est traversée par le sang à un débit sanguin prédéterminé $Q_b$, et en une chambre de liquide de dialyse, qui est traversée par un liquide de dialyse à un débit de liquide de dialyse $Q_d$, dans lequel le débit du liquide de dialyse $Q_d$ ou le débit sanguin $Q_b$ est déterminé en fonction du débit sanguin $Q_b$ ou du débit de liquide de dialyse $Q_d$ prédéterminé, **caractérisé en ce que** en fonction du débit sanguin $Q_b$ le débit du liquide de dialyse $Q_d$ ou en fonction du débit du liquide de dialyse $Q_d$ le débit sanguin $Q_b$ est déterminé, dans le cas de leur augmentation d'une valeur déterminée, l'augmentation d'une grandeur caractéristique pour l'efficacité du traitement sanguin ne dépassant pas une valeur déterminée.

**12.** Procédé selon la revendication 11, **caractérisé en ce que** la grandeur caractéristique pour le traitement de dialyse est la clairance K du traitement de dialyse.

**13.** Procédé selon la revendication 11 ou 12, **caractérisé en ce que** le débit du liquide de dialyse $Q_d$ ou le débit sanguin $Q_b$, dans le cas de leur augmentation d'une valeur déterminée, ne dépasse pas l'augmentation d'une grandeur caractéristique pour l'efficacité du traitement sanguin d'une valeur déterminée, déterminée en fonction d'une grandeur caractéristique pour le dialyseur.

**14.** Procédé selon la revendication 13, **caractérisé en ce que** la grandeur caractéristique pour le dialyseur est le coefficient de transfert de masse KOA du dialyseur.

**15.** Procédé selon l'une des revendications 11 à 14, **caractérisé en ce que** différents modes de traitement ayant différentes efficacités du traitement sanguin sont prédéterminés, dans lequel, pour le mode de traitement respectif, le débit du liquide de dialyse $Q_d$ ou le débit sanguin $Q_b$ est déterminé, dans le cas de leur augmentation d'une valeur déterminée, l'augmentation de la grandeur caractéristique pour l'efficacité du traitement sanguin ne dépassant pas une valeur attribuée au mode de traitement respectif.

**16.** Procédé selon l'une des revendications 11 à 15, **caractérisé en ce que** le débit du liquide de dialyse $Q_d$ ou le débit sanguin $Q_b$ est calculé selon une équation mathématique.

**17.** Procédé selon la revendication 16, **caractérisé en ce que** l'équation mathématique pour le calcul du débit du liquide de dialyse $Q_d$ est un polynôme de degrés multiples.

# Fig. 1

Fig. 2

Fig. 3

Fig. 4

Fig. 5

Fig. 6

Fig. 7

## QD:K = 10:1

Fig. 8

## QD:K = 5:1

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5100554 A
- DE 69531137 T2
- WO 9532010 A
- US 5092836 A

**In der Beschreibung aufgeführte Nicht-Patentliteratur**

- **J.E. SIEGDELL ; B. TERSTEEGEN.** *Artificial Organs,* 1986, vol. 10 (3), 219-225